# EUROPÄISCHE PATENTSCHRIFT

(11) **EP 0 444 264 B1**
(45) Veröffentlichungstag und Bekanntmachung des Hinweises auf die Patenterteilung: **27.10.1993**
(21) Anmeldenummer: 90123930.1
(22) Anmeldetag: 12.12.1990
(51) Int. Cl.: B01J 31/32, B01J 23/36, B01J 23/84, C07C 67/475, C07C 6/04, C07C 11/02, C07C 69/533

(54) **Katalysatoren für die Metathese von Olefinen und funktionalisierten Olefinen**
Catalysts for the metathesis of olefins and functionalised olefins
Catalyseurs pour la métathèse d'oléfines et d'oléfines fonctionnalisées

(30) Priorität: 02.03.1990 DE 4006540
(43) Veröffentlichungstag der Anmeldung: 04.09.1991
(73) Patentinhaber: HÜLS AKTIENGESELLSCHAFT, 45764 Marl (DE)
(72) Erfinder: Warwel, Siegfried, Prof. Dr., W-5100 Aachen (DE); Jägers, Hans-Gerd, Dr., W-4390 Gladbeck (DE); Deckers, Andreas, Dr., W-6700 Ludwigshafen (DE)

(56) Entgegenhaltungen:
- DE-A- 3 229 419
- FR-A- 2 521 872
- CHEMICAL ABSTRACTS, Band 105, Nr. 17, 27. Oktober 1986, Columbus, Ohio, USA XU, XIAODING et al. "Rhenium (VII) oxide/alumina-boron oxide metathesis catalysts" Seite 636, Spalte 2, Zusammenfassung-Nr. 152 416q
- JOURNAL OF ORGANOMETALLIC CHEMISTRY, Band 255, 1983, Lausanne R.H.A. BOSMA et al. "Heterogenous metathesis of unsaturated esters using a rhenium-base catalyst" Seiten 159-171
- CHEMICAL ABSTRACTS, Band 105, Nr. 18, 3. November 1986, Columbus, Ohio, USA XU, XIAODING et al. "Rhenium oxide-containing metathesis catalysts: a study of temperature programmed reduc- tion and thermal analysis" Seite 422, Spalte 2, Zusammenfassung-Nr. 159 447j

## Beschreibung

Die Erfindung betrifft heterogene Katalysatoren auf einem Al₂O₃-SiO₂-Träger für die Metathese von Olefinen und funktionalisierten Olefinen.

Die Metathese olefinischer Kohlenwasserstoffe dient in der Technik zur Herstellung von speziellen Olefinen, Di- und Polyenen sowie ungesättigten Polymeren. Auch Olefine mit funktionellen Gruppen unterliegen der Metathesereaktion, sofern geeignete Katalysatoren zur Anwendung kommen. Von besonderer Bedeutung ist hierbei die Metathese ungesättigter Fettsäuremethylester, die durch Umesterung nativer Fette und Öle mit Methanol im großindustriellen Maßstab produziert werden und damit als Ausgangsverbindungen wirtschaftlich zur Verfügung stehen. Durch Metathese dieser Ester eröffnen sich neue einfache Zugänge zu chemisch-technisch wichtigen Zwischenprodukten für die industrielle Herstellung von Tensiden, Kunststoffen, Kunststoffweichmachern, Schmierölen sowie einer ganzen Palette von Feinchemikalien.

Nach Bosma et al, Journal of Organometallic Chemistry 255 (1983), 159 - 71, kann die Metathese von ungesättigten Estern an einem Re₂O₇/Al₂O₃-Katalysator, der mit einer zinnorganischen Verbindung der allgemeinen Formel SnR₄ aktiviert ist, durchgeführt werden. Zur Herstellung des Katalysators wird gamma-Al₂O₃ mit Ammoniumperrhenat imprägniert.

Warwel, Erdöl-Erdgas-Kohle 103 (1987), 238 - 45, beschreibt industrielle Metatheseprozesse, bei denen vorwiegend Re₂O₇/Al₂O₃-, CoO-MoO₃/Al₂O₃- und WO₃/SiO₂-Katalysatoren verwendet werden. Danach ist nur der Re₂O₇/Al₂O₃-Katalysator bereits bei Raumtemperatur aktiv.

Nach FR 2 521 872 kann die Metathese von funktionalisierten Olefinen an Re₂O₇/Al₂O₃-SiO₂-Katalysatoren durchgeführt werden. Der Träger enthält dabei vorzugsweise nur 10 bis 30 % SiO₂. Als Aktivatoren dienen hier organische Bleiverbindungen der allgemeinen Formel PbR₄.

In NL-A-84 03 051 wird die Metathese vorzugsweise an SiO₂-reichen Trägerkatalysatoren durchgeführt. Die Re₂O₇/Al₂O₃-SiO₂-Katalysatoren enthalten im Träger vorzugsweise 65 bis 90 % SiO₂. Die Aktivatoren sind hier vorzugsweise Zinntetraethyl und -tetrabutyl.

Für die praktische Anwendung der Katalysatoren auf die Metathese, insbesondere auf die Metathese von ungesättigten Estern, besteht das zentrale Problem, daß die Katalysatoren in hohen Konzentrationen eingesetzt werden müssen. Es gibt vor allem noch keine Katalysatoren, die eine wirtschaftliche Nutzung der Metathese ungesättigter Fettsäureester erlauben.

Es stellte sich daher die Aufgabe, wirksamere Katalysatoren für die Metathese von Olefinen und von funktionalisierten Olefinen zu entwickeln. Vor allem sollten die Katalysatoren bei der Metathese von ungesättigten Fettsäureestern höhere Umsätze und höhere Ausbeuten ermöglichen.

Die Aufgabe wird erfindungsgemäß durch die Verwendung von B₂O₃-Re₂O₇/Al₂O₃-SiO₂-Katalysatoren gelöst.

In diesen Katalysatoren sind Boroxid und Rheniumoxid die eigentlich katalytisch wirksamen Verbindungen.

Das Alumosilikat Al₂O₃-SiO₂ ist der Träger. Überraschenderweise hat sich herausgestellt, daß die katalytische Wirkung auf einem Alumosilikatträger höher ist als auf einem SiO₂- oder einem Al₂O₃-Träger. Im Träger liegt der SiO₂-Anteil vorzugsweise bei 20 bis 50 Gewichtsprozent, wobei ein Gehalt von 35 bis 50 Gewichtsprozent besonders bevorzugt wird.

Der B₂O₃-Re₂O₇-Anteil liegt bei 2 bis 30 Gewichtsprozent. Dabei kann der B₂O₃-Gehalt 1 bis 20 Gewichtsprozent betragen. Der Gehalt liegt vorzugsweise bei 2 bis 10 Gewichtsprozent. Der Re₂O₇-Gehalt schwankt im Bereich von 1 bis 20 Gewichtsprozent. 2 bis 15 Gewichtsprozent Re₂O₇ werden bevorzugt. Die Gewichtsprozente beziehen sich jeweils auf den Alumosilikat-Träger.

Bei der Herstellung der Katalysatoren imprägniert man im allgemeinen den Al₂O₃-SiO₂-Träger mit einem Salz oder einer anderen Verbindung der katalytisch aktiven Elemente. So kann man den Träger gleichzeitig mit Borsäure und Ammoniumperrhenat imprägnieren. Nach Trocknung und Erhitzen entstehen der oxidische Katalysator. Man kann aber auch nacheinander zuerst Borsäure auftragen, entwässern und in das Oxid überführen und danach mit dem Rheniumsalz imprägnieren, dieses trocknen und ebenfalls in das Oxid überführen.

Die erfindungsgemäßen Katalysatoren können als Pulver, Granulat oder als Wabenkörper eingesetzt werden. In Pulverform werden die Katalysatoren vorzugsweise verwendet.

Die B₂O₃-Re₂O₇/Al₂O₃-SiO₂-Katalysatoren sind signifikant wirksamer für Metathesereaktionen als bekannte Katalysatoren, die kein B₂O₃ enthalten oder anstelle eines Al₂O₃-SiO₂-Trägers einen gamma-Al₂O₃-Träger aufweisen.

Bei der Metathese von Olefinkohlenwasserstoffen wird neben den erfindungsgemäßen Katalysatoren kein Aktivator benötigt. Bei der Metathese von funktionalisierten Olefinen oder bei der Co-Metathese von Olefinen und funktionalisierten Olefinen ist der Einsatz eines Aktivators jedoch notwendig.

Funktionalisierte Olefine im Sinne der Erfindung sind ungesättigte Ester, Ether, Halogen- und Stickstoffverbindungen, Aldehyde, Ketone sowie derivatisierte Alkohole und derivatisierte Carbonsäuren. Vorzugsweise werden ungesättigte Carbonsäureester eingesetzt.

Geeignete Aktivatoren sind zinnorganische Verbindungen, wobei man Zinntetraalkyl der Formel SnR₄, wobei R Alkyl mit 1 bis 8 Kohlenstoffatomen ist, bevorzugt. Beispiele für R sind Methyl, Ethyl, Isopropyl und n-Butyl.

Der Aktivator wird vorzugsweise in solchen Mengen eingesetzt, daß das molare Verhältnis von Re₂O₇ : SnR₄ bei 5 : 1 bis 1 : 5 liegt. Im besonderen wird ein molares Verhältnis von 2 : 1 bis 1 : 2 bevorzugt.

Mit der Kombination aus B₂O₃-Re₂O₇/Al₂O₃-SiO₂-Katalysator und SnR₄-Aktivator können sowohl reine Olefinkohlenwasserstoffe als auch funktionalisierte Olefine, z. B. ungesättigte Carbonsäureester, metathetisiert werden, wobei im letzteren Fall selbst bei molaren Verhältnissen von Re₂O₇ : Ester von 1 : 1 000 noch hohe Umsätze erzielt werden. Die Metathesereaktionen können als Homo-Metathese, als Co-Metathese (Einsatz zweier unterschiedlicher olefinischer Verbindungen) und bei Cycloolefinen als Substrat als metathetische, ringöffnende Polymerisation durchgeführt werden. Die Metathesereaktionen werden bevorzugt bei Raumtemperatur durchgeführt, was aus Gründen der Energieeinsparung vorteilhaft ist. Die Anwendung tieferer oder höherer Temperatur ist jedoch auch möglich.

Die Erfindung wird durch die nachfolgenden Beispiele näher erläutert. Dabei werden erfindungsgemäße Beispiele durch Zahlen und Vergleichsbeispiele durch Buchstaben gekennzeichnet.

### Beispiel 1

Darstellung des Katalysators B₂O₃-Re₂O₇/Al₂O₃-SiO₂

In einem 250-ml-Einhalskolben werden 0,55 g NH₄ReO₄ und 1,17 g H₃BO₃ in 135 ml Dioxan und 15 ml H₂O gelöst. Nach der Zugabe von 10 g zuvor bei 500 °C im Luftstrom calciniertem Alumosilikat mit 40 Gewichtsprozent SiO₂ wird das gesamte Gemisch über Nacht unter Rückfluß erhitzt. Anschließend wird das Lösemittel abdestilliert und der Feststoff bei 130 °C im Wasserstrahlvakuum vorgetrocknet. Der vorgetrocknete Kontakt wird im Luftstrom auf 500 °C erhitzt und 16 Stunden bei dieser Temperatur belassen. Dann läßt man ihn unter Argon auf Raumtemperatur abkühlen. Durch Elementaranalyse wird ein Gehalt von 4,4 Gewichtsprozent Re₂O₇ und 5,8 Gewichtsprozent B₂O₃ festgestellt.

### Beispiel 2

Co-Metathese von 10-Undecensäuremethylester mit 4-Octen
Katalysator: B₂O₃-Re₂O₇/Al₂O₃-SiO₂ + Sn(n-C₄H₉)₄

In einem zuvor ausgeheizten und mit Argon befüllten 50-ml-Schlenkgefäß mit Magnetrührkern werden 0,5 g des in Beispiel 1 dargestellten Katalysators (mit 0,045 mmol Re₂O₇) vorgelegt und mit 0,57 g (0,54 ml) einer SnBu₄-Lösung (0,1molar in Chlorbenzol, mit 0,054 mmol SnBu₄) sowie 1 ml Chlorbenzol versetzt. Anschließend werden 8,06 g (11,23 ml) 4-Octen (72 mmol) und 7,13 g (8,03 ml) 10-Undecensäuremethylester (36 mmol) zugegeben, wodurch ein molares Verhältnis der einzelnen Komponenten wie folgt gegeben ist:
Re₂O₇ : SnBu₄ : C₁₁-Ester : 4-Octen = 1 : 1,2 : 800 : 1 600

Nach 2stündigem Rühren bei Raumtemperatur wird mittels einer Spritze eine Probe entnommen und nach Zugabe einiger Tropfen Methanol zur Zerstörung etwaiger Katalysatorreste gaschromatographisch analysiert.

Gemäß der Reaktionsgleichung 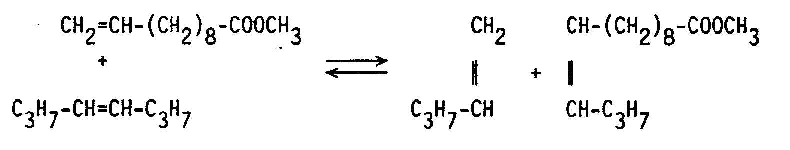
enthält die Reaktionsmischung neben den Ausgangsverbindungen 1-Penten und 10-Tetradecensäureester. Durch Homo-Metathese von 10-Undecensäureester wird in geringem Maße gemäß 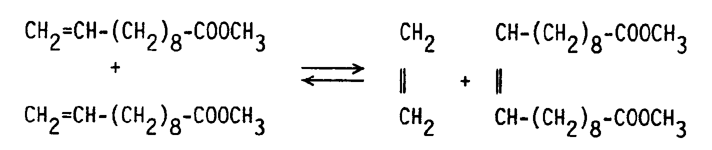
auch der ungesättigte C₂₀-Diester gebildet.

Der Umsatz des eingesetzten 10-Undecensäureesters beträgt 77 %. Von den neugebildeten Estern macht der C₁₄-Monoester 87 Gewichtsprozent und der C₂₀-Diester 13 Gewichtsprozent aus.

### Vergleichsbeispiel A

Co-Metathese von 10-Undecensäuremethylester mit 4-Octen
Katalysator: Re₂O₇/Al₂O₃-SiO₂ + Sn(n-C₄H₉)₄

Der Versuch aus Beispiel 2 wird wiederholt, jedoch wird ein B₂O₃-freier Katalysator eingesetzt. Gegenüber Beispiel 2 sinkt der Reaktionsumsatz auf 59 % ab.

### Vergleichsbeispiel B

Co-Metathese von 10-Undecensäuremethylester mit 4-Octen
Katalysator: B₂O₃-Re₂O₇/gamma-Al₂O₃ + Sn(n-C₄H₉)₄

Der Versuch aus Beispiel 2 wird wiederholt, jedoch wird ein SiO₂-freier Katalysator eingesetzt (Austausch von Alumosilikat gegen gamma-Al₂O₃). Gegenüber Beispiel 2 sinkt der Reaktionsumsatz auf 30 % ab.

### Beispiel 3

Co-Metathese von Ölsäuremethylester mit 4-Octen
Katalysator: B₂O₃-Re₂O₇/Al₂O₃-SiO₂ + Sn(n-C₄H₉)₄

In einem zuvor ausgeheizten und mit Argon befüllten 50-ml-Schlenkgefäß mit Magnetrührkern werden 0,5 g des in Beispiel 1 dargestellten Katalysators (mit 0,045 mmol Re₂O₇) vorgelegt und mit 0,57 g (0,54 ml) einer SnBu₄-Lösung (0,1molar in Chlorbenzol, mit 0,054 mmol SnBu₄) sowie 1 ml Chlorbenzol versetzt. Anschließend werden 8,06 g 4-Octen (72 mmol) und 10,63 g Ölsäuremethylester (36 mmol) zugegeben, wodurch ein molares Verhältnis der einzelnen Komponenten wie folgt gegeben ist:
Re₂O₇ : SnBu₄ : Ölsäuremethylester : 4-Octen = 1 : 1,2 : 800 : 1 600

Nach einer Reaktionszeit von 2 Stunden bei Raumtemperatur wird eine Probe entnommen und nach Zugabe einiger Tropfen Methanol zur Zerstörung etwaiger Katalysatorreste gaschromatographisch analysiert.

Gemäß der Reaktionsgleichung 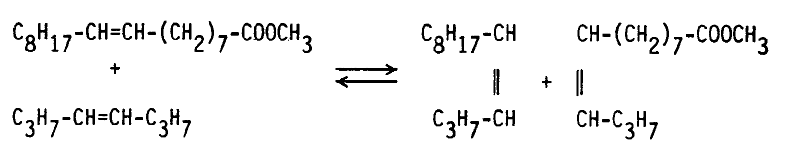
enthält die Reaktionsmischung jetzt 4-Tridecen und 9-Tridecensäuremethylester. Durch Homo-Metathese des Ölsäuremethylesters gemäß 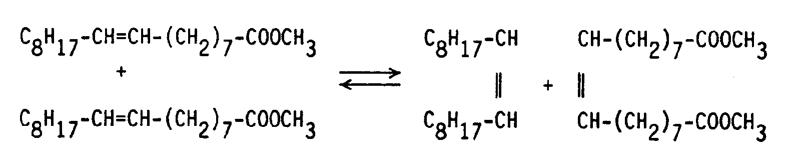
werden ferner geringe Mengen 9-Octadecen und 9-Octadecendisäuredimethylester gebildet.

Es wird ein Reaktionsumsatz des eingesetzten Ölsäuremethylesters von 81 % festgestellt. Die neugebildeten Ester setzen sich zu 89 Gewichtsprozent aus dem ungesättigten C₁₃-Monoester und zu 11 Gewichtsprozent aus dem ungesättigten C₁₈-Diester zusammen.

### Vergleichsbeispiel C

Co-Metathese von Ölsäuremethylester mit 4-Octen
Katalysator: Re₂O₇/Al₂O₃-SiO₂ + Sn(n-C₄H₉)₄

Der Versuch von Beispiel 3 wird wiederholt, jedoch wird ein B₂O₃-freier Katalysator eingesetzt. Der Umsatz sinkt auf 40 % ab.

### Vergleichsbeispiel D

Co-Metathese von Ölsäuremethylester mit 4-Octen
Katalysator: B₂O₃-Re₂O₇/gamma-Al₂O₃ + Sn(n-C₄H₉)₄

Der Versuch von Beispiel 3 wird wiederholt, jedoch wird ein SiO₂-freier Katalysator eingesetzt. Außerdem werden die Katalysator- und die Aktivatorkonzentration nahezu verdoppelt. Das molare Verhältnis liegt jetzt bei Re₂O₇ : SnBu₄ : Ölsäuremethylester : 4-Octen = 1 : 1,5 : 500 : 1 000. Trotzdem geht der Umsatz auf 27 % zurück.

### Beispiel 4

Homo-Metathese von 10-Undecensäuremethylester
Katalysator: B₂O₃-Re₂O₇/Al₂O₃-SiO₂ + Sn(n-C₄H₉)₄

In einem zuvor ausgeheizten und mit Argon befüllten 50-ml-Schlenkgefäß mit Rückflußkühler, an den ein Blasenzähler angeschlossen ist, werden 0,5 g des in Beispiel 1 dargestellten Katalysators vorgelegt und mit 0,57 g (054 ml) einer SnBu₄-Lösung (0,1molar in Chlorbenzol) sowie 1 ml Chlorbenzol versetzt. Anschließend werden 3,57 g 10-Undecensäuremethylester (18 mmol) zugegeben, wodurch ein molare Verhältnis der einzelnen Komponenten von
Re₂O₇ : SnBu₄ : C₁₁-Ester = 1 : 1,2 : 400 gegeben ist.

Nach 2 Stunden bei Raumtemperatur wird gaschromatographisch ein Umsatz von 49 % festgestellt. Bei Wiederholung des Versuchs bei 80 °C wird nach 2 Stunden ein Umsatz von 60 % festgestellt. Bei beiden Versuchen wird gemäß 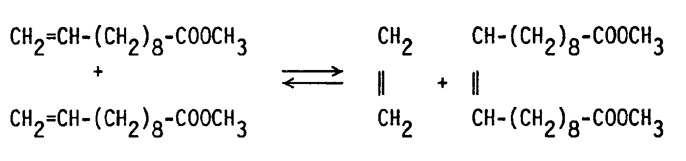
neben Ethen ausschließlich 10-Eicosendisäuredimethylester gebildet.

### Beispiel 5

Homo-Metathese von Ölsäuremethylester
Katalysator: B₂O₃-Re₂O₇/Al₂O₃-SiO₂ + Sn(n-C₄H₉)₄

In einem zuvor ausgeheizten und mit Argon befüllten 50-ml-Schlenkgefäß mit Magnetrührkern werden 0,5 g des in Beispiel 1 dargestellten Katalysators vorgelegt und mit 0,57 g (0,54 ml) einer SnBu₄-Lösung (0,1molar in Chlorbenzol) sowie 1 ml Chlorbenzol versetzt. Anschließend werden 5,32 g Ölsäuremethylester (18 mmol) zugegeben, wodurch ein molares Verhältnis der einzelnen Komponenten von Re₂O₇ : SnBu₄ : Ölsäuremethylester = 1 : 1,2 : 400 gegeben ist.

Nach 2 Stunden bei 80 °C wird ein Umsatz des Ölsäuremethylesters von 45 % zu 9-Octadecen und 9-Octadecendisäuredimethylester gemäß 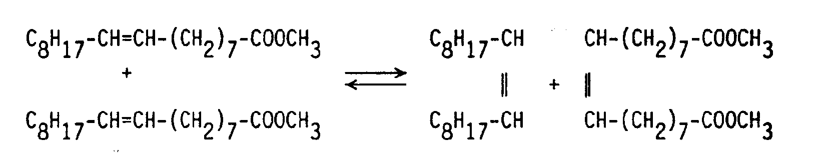
erzielt.

### Beispiel 6

Co-Metathese von 4-Octen mit 5-Decen
Katalysator: B₂O₃-Re₂O₇/Al₂O₃-SiO₂ + Sn(n-C₄H₉)₄

In einem zuvor ausgeheizten und mit Argon befüllten 50-ml-Schlenkgefäß mit Magnetrührkern werden 1,6 g des in Beispiel 1 dargestellten Katalysators (mit 0,146 mmol Re₂O₇) vorgelegt und mit 1,7 ml einer 0,1molaren SnBu₄-Lösung in Chlorbenzol (mit 0,173 mmol SnBu₄) sowie 1 ml Chlorbenzol versetzt. Anschließend werden 13,1 g 4-Octen (116,8 mmol) sowie 16,4 g 5-Decen (116,8 mmol) zugegeben, wodurch ein molaren Verhältnis der einzelnen Komponenten von
Re₂O₇ : SnBu₄ : 4-Octen : 5-Decen = 1 :1,2 : 800 : 800 gegeben ist.

Nach 2 Stunden bei Raumtemperatur wird eine Probe entnommen und gaschromatographisch analysiert. Gemäß der Reaktionsgleichung 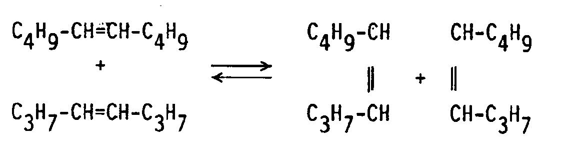
enthält die Reaktionsmischung neben den Einsatzstoffen auch 4-Nonen. Der Umsatz der eingesetzten Olefine beträgt 50 %, die Selektivität zu 4-Nonen beträgt 98 %.

### Beispiel 7

Co-Metathese von 4-Octen mit 5-Decen
Katalysator: B₂O₃-Re₂O₇/Al₂O₃-SiO₂

Der Versuch aus Beispiel 6 wird wiederholt, jedoch wird kein zinnorganischer Aktivator verwendet. Auch bei diesem Versuch wird gaschromatographisch ein Umsatz von 50 % festgestellt bei einer Selektivität zu 4-Nonen von 90 %.

### Beispiel 8

Homo-Metathese von 1-Octen
Katalysator: B₂O₃-Re₂O₇/Al₂O₃-SiO₂ + Sn(n-C₄H₉)₄

Der Versuch aus Beispiel 6 wird wiederholt, jedoch wird anstelle von 4-Octen/5-Decen nunmehr 1-Octen eingesetzt und das molare Verhältnis der einzelnen Komponenten auf Re₂O₇ : SnBu₄ : 1-Octen = 1 : 8 : 12 000 eingestellt.

Nach 3 Stunden bei Raumtemperatur wird gaschromatographisch ein Umsatz von 1-Octen zu Ethylen und 7-Tetradecen von 42 % bei einer Selektivität von 86 % festgestellt.

## Patentansprüche

1. B₂O₃-Re₂O₇/Al₂O₃-SiO₂-Katalysatoren für die Metathese von Olefinen und funktionalisierten Olefinen.

2. Katalysatoren nach Anspruch 1, die einen B₂O₃-Re₂O₇-Gehalt von 2 bis 30 Gewichtsprozent, bezogen auf den Al₂O₃-SiO₂-Anteil, aufweisen.

3. Katalysatoren nach Anspruch 1, bei denen der Re₂O₇-Gehalt, bezogen auf den Al₂O₃-SiO₂-Anteil, bei 1 bis 20 Gewichtsprozent, vorzugsweise bei 2 bis 15 Gewichtsprozent, liegt.

4. Katalysatoren nach Anspruch 1, bei denen der B₂O₃-Gehalt, bezogen auf den Al₂O₃-SiO₂-Anteil, bei 1 bis 20 Gewichtsprozent, vorzugsweise bei 2 bis 10 Gewichtsprozent, liegt.

5. Katalysatoren nach Anspruch 1, die 20 bis 50 Gewichtsprozent SiO₂, bezogen auf den Gehalt an Al₂O₃-SiO₂, enthalten.

6. Katalysatoren nach Anspruch 5, die 35 bis 50 Gewichtsprozent SiO₂, bezogen auf Al₂O₃-SiO₂, aufweisen.

7. Katalysatoren nach Anspruch 1,
dadurch gekennzeichnet,
daß sie zusätzlich eine zinnorganische Verbindung enthalten.

8. Katalysatoren nach Anspruch 7,
dadurch gekennzeichnet,
daß sie SnR₄, wobei R eine Alkylgruppe mit 1 bis 8 Kohlenstoffatomen ist, enthalten.

9. Katalysatoren nach Anspruch 8,
dadurch gekennzeichnet,
daß das molare Verhältnis von Re₂O₇ : SnR₄ bei 5 : 1 bis 1 : 5, vorzugsweise bei 2 : 1 bis 1 : 2, liegt.

10. Katalysatoren nach Anspruch 1,
dadurch gekennzeichnet,
daß die funktionalisierten Olefine ungesättigte Carbonsäureester sind.

## Claims

1. A B₂O₃-Re₂O₇/Al₂O₃-SiO₂ catalyst for the metathesis of olefins and functionalised olefins.

2. A catalyst according to Claim 1, which has a B₂O₃-Re₂O₇ content of from 2 to 30 per cent by weight, based on the Al₂O₃-SiO₂ portion.

3. A catalyst according to Claim 1, in which the Re₂O₇ content, based on the Al₂O₃-SiO₂ portion, is from 1 to 20 per cent by weight, preferably from 2 to 15 per cent by weight.

4. A catalyst according to Claim 1, in which the B₂O₃ content, based on the Al₂O₃-SiO₂ portion, is from 1 to 20 per cent by weight, preferably from 2 to 10 per cent by weight.

5. A catalyst according to Claim 1, which contains from 20 to 50 per cent by weight of SiO₂, based on the content of Al₂O₃-SiO₂.

6. A catalyst according to Claim 5, which contains from 35 to 50 per cent by weight of SiO₂, based on Al₂O₃-SiO₂.

7. A catalyst according to Claim 1, characterised in that it additionally contains an organotin compound.

8. A catalyst according to Claim 7, characterised in that it contains SnR₄, in which R is an alkyl group having from 1 to 8 carbon atoms.

9. A catalyst according to Claim 8, characterised in that the molar ratio of Re₂O₇ : SnR₄ is from 5 : 1 to 1 : 5, preferably from 2 : 1 to 1 : 2.

10. A catalyst according to Claim 1, characterised in that the functionalised olefins are unsaturated carboxylic esters.

## Revendications

1. Catalyseurs à base d'un mélange de B ₂0₃-Re₂0₇ et de Al₂0₃-Si0₂ pour la metathèse d'oléfines et d'oléfines fonctionnalisées.

2. Catalyseurs selon la revendication 1, caractérisés par le fait qu'ils présentent une teneur en B₂0₃-Re₂0₇ de 2 à 30 % en poids, relativement à la fraction de Al₂0₃-Si0₂.

3. Catalyseurs selon la revendication 1, caractérisés par le fait que la teneur en Re₂0₇, relativement à la fraction de Al₂0₃-Si0₂, se situe à une valeur de 1 à 20 % en poids, de préférence à une valeur de 2 à 15 % en poids.

4. Catalyseurs selon la revendication 1, caractérisés par le fait que la teneur en B₂0₃, relativement à la fraction de Al₂0₃-Si0₂, se situe à une valeur de 1 à 20 % en poids, de préférence à une valeur de 2 à 10 % en poids.

5. Catalyseurs selon la revendication 1, caractérisés par le fait qu'ils renferment de 20 à 50 % en poids de Si0₂, relativement à la teneur en Al₂0₃-SiO₂ .

6. Catalyseurs selon la revendication 5, caractérisés par le fait qu'ils presentent de 35 à 50 % en poids de Si0₂, relativement à Al₂0₃-Si0₂.

7. Catalyseurs selon la revendication 1, caractérises par le fait qu'ils renferment complémentairement un composé organique de l'étain.

8. Catalyseurs selon la revendication 7, caractérisés par le fait qu'ils renferment SnR₄, où R est un groupe alkyle comportant de 1 à 8 atomes de carbone.

9. Catalyseurs selon la revendication 8, caractérisés par le fait que le rapport molaire de Re₂0₇ à SnR₄ se situe à une valeur de 5 : 1 à 1 : 5, de préférence de 2 : 1 à 1 : 2.

10. Catalyseurs selon la revendication 1, caractérisés par le fait que les oléfines fonctionnalisées sont des esters insaturés d'acide carboxylique.
